# EUROPÄISCHE PATENTSCHRIFT

(11) **EP 1 802 622 B1**
(45) Veröffentlichungstag und Bekanntmachung des Hinweises auf die Patenterteilung: **23.04.2008**
(21) Anmeldenummer: 05792312.0
(22) Anmeldetag: 28.09.2005
(51) Int. Cl.: C07D 471/06, C09B 5/62, C08K 5/3437

(54) **HALOGENIERUNG VON RYLENCARBONSÄUREUREIMIDEN MIT ELEMENTAREM HALOGEN IN EINEM ZWEIPHASENGEMISCH BESTEHEND AUS ORGANISCHEM LÖSUNGSMITTEL UND WASSER, WOBEI DER SICH BILDENDE HALOGENWASSERSTOFF DEM ORGANISCHEN LÖSUNGSMITTEL KONTINUIERLICH ENTZOGEN WIRD**
HALOGENATION OF RYLEN-CARBOXIMIDES WITH ELEMENTARY HALOGEN IN A TWO-PHASE MIXTURE COMPRISING AN ORGANIC SOLVENT AND WATER, WHEREIN FORMED HALOGEN HYDROCIDE IS CONTINUOUSLY REMOVABLE FROM THE ORGANIC SOLVENT
HALOGENATION DE RYLENE-CARBOXIMIDES A HALOGENE ELEMENTAIRE DANS UN MELANGE A DEUX PHASES COMPRENANT UN SOLVANT ORGANIQUE ET DE L'EAU, AVEC PRELEVEMENT EN CONTINU, A PARTIR DU SOLVANT ORGANIQUE, DE L'HYDRACIDE HALOGENE FORME

(30) Priorität: 05.10.2004 DE 102004048729
(43) Veröffentlichungstag der Anmeldung: 04.07.2007
(73) Patentinhaber: BASF SE, 67056 Ludwigshafen (DE)
(72) Erfinder: KÖNEMANN, Martin, 68163 Mannheim (DE); PSCHIRER, Neil, Gregory, 55116 Mainz (DE)
(86) Internationale Anmeldenummer: PCT/EP2005/010490
(87) Internationale Veröffentlichungsnummer: WO 2006/037539

(56) Entgegenhaltungen:
- WO-A-03/104232
- DD-A1- 159 066
- SU-A1- 392 064

## Beschreibung

Die vorliegende Erfindung betrifft ein neues Verfahren zur Herstellung von halogenierten Rylencarbonsäureimiden der allgemeinen Formel I 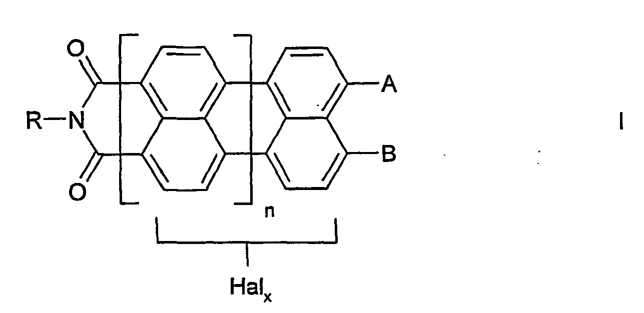 in der die Variablen folgende Bedeutung haben:
- A, B: gemeinsam ein Imidrest der Formel 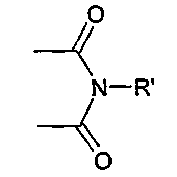 oder für den Fall, daß n gleich 1 ist, A und B auch beide Hal oder ein Rest Hal und der andere Rest Wasserstoff;
- R, R': unabhängig voneinander:
Wasserstoff;
(1) C₁-C₃₀-Alkyl, dessen Kohlenstoffkette durch eine oder mehrere Gruppierungen -O-, -S-, -NR¹- -N=CR¹- -C≡C-, -CR¹=CR¹-, -CO-, -SO- und/oder -SO₂- unterbrochen sein kann und das ein- oder mehrfach substituiert sein kann durch:
   (i) C₁-C₁₂-Alkoxy, C₁-C₆-Alkylthio, -C≡CR¹, -CR¹=CR¹₂, Hydroxy, Mercapto, Halogen, Cyano, Nitro, -NR²R³, -NR²COR³, -CONR²R³, -SO₂NR²R³, -COOR², -SO₃R², -PR²R³ und/oder -POR²R³;
   (ii) Aryl oder Hetaryl, an das weitere gesättigte oder ungesättigte 5- bis 7-gliedrige Ringe, deren Kohlenstoffgerüst durch eine oder mehrere Gruppierungen -O-, -S-, -NR¹-, -N=CR¹-, -CR¹=CR¹, -CO-, -SO- und/oder -SO₂- unterbrochen sein kann, anneliert sein können, wobei das gesamte Ringsystem ein- oder mehrfach substituiert sein kann durch: C₁-C₁₈-Alkyl, C₁-C₁₂-Alkoxy, C₁-C₆-Alkylthio, -C≡CR¹, -CR¹=CR¹₂, Hydroxy, Mercapto, Halogen, Cyano, Nitro, -NR²R³, -NR²COR³, -CONR²R³, -SO₂NR²R³ -COOR ², -SO₃R², -PR²R³, -POR²R³, Aryl und/oder Hetaryl, das jeweils durch C₁-C₁₈-Alkyl, C₁-C₁₂-Alkoxy, Hydroxy, Mercapto, Halogen, Cyano, Nitro, -NR²R³, -NR²COR³, -CONR²R³, -SO₂NR²R³, -COOR², -SO₃R ², -PR²R³ und/oder -POR²R³ substituiert sein kann;
   (iii) C₃-C₈-Cycloalkyl, dessen Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann und an das weitere gesättigte oder ungesättigte 5- bis 7-gliedrige Ringe, deren Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann, anneliert sein können, wobei das gesamte Ringsystem ein- oder mehrfach substituiert sein kann durch: C₁-C₁₈-Alkyl, C₁-C₁₂-Alkoxy, C₁-C₆-Alkylthio, -C≡CR¹, -CR¹=CR¹, Hydroxy, Mercapto, Halogen, Cyano, Nitro, -NR²R³, -NR²COR³, -CONR²R³, -SO₂NR²R³, -COOR², -SO₃R² -PR²R³ und/oder -POR²R³;
   (iv) einen Rest -U-Aryl, der ein- oder mehrfach durch die vorstehenden, als Substituenten für die Arylreste (ii) genannten Reste substituiert sein kann, wobei U eine Gruppierung -O-, -S-, -NR¹-, -CO-, -SO- oder -SO₂- bedeutet;
(2) C₃-C₈-Cycloalkyl, an das weitere gesättigte oder ungesättigte 5- bis 7-gliedrige Ringe, deren Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann, anneliert sein können, wobei das gesamte Ringsystem ein- oder mehrfach sein kann durch: die Reste (i), (ii), (iii), (iv) und/oder (v) C₁-C₃₀-Alkyl, dessen Kohlenstoffkette durch eine oder mehrere Gruppierungen -O-, -S-, -NR¹-, -N=CR¹-, -C≡C-, -CR¹=CR¹-, -CO-, -SO- und/oder -SO₂- unterbrochen sein kann und das ein- oder mehrfach substituiert sein kann durch: C₁-C₁₂-Alkoxy, C₁-C₆-Alkylthio, -C≡CR¹, -CR¹=CR¹₂, Hydroxy, Mercapto, Halogen, Cyano, Nitro, -NR²R³, -NR²COR³, -CONR²R³, -SO₂NR²R³, -COOR², -SO₃R², -PR²R³ -POR²R³, Aryl und/oder gesättigtes oder ungesättigtes C₄-C₇-Cycloalkyl, dessen Kohlenstoffgerüst durch eine oder mehrere Gruppierungen -O-, -S-, -NR¹-, -N=CR¹- -CR¹=CR¹-, -CO-, -SO- und/oder -SO₂- unterbrochen sein kann, wobei die Aryl- und Cycloalkylreste jeweils ein- oder mehrfach durch C₁-C₁₈-Alkyl und/oder die vorstehenden, als Substituenten für Alkyl genannten Reste substituiert sein können;
(3) Aryl oder Hetaryl, an das weitere gesättigte oder ungesättigte 5- bis 7-gliedrige Ringe, deren Kohlenstoffgerüst durch eine oder mehrere Gruppierungen -O-, -S-, -NR¹-, -N=CR¹-, -C≡C-, -CR¹=CR¹-, -CO-, -SO- und/oder -SO₂- unterbrochen sein kann, anneliert sein können, wobei das gesamte Ringsystem substituiert sein kann durch die Reste (i), (ii), (iii), (iv), (v) und/oder Aryl- und/oder Hetarylazo, das jeweils durch C₁-C₁₀-Alkyl, C₁-C₆-Alkoxy und/oder Cyano substituiert sein kann;
   Wasserstoff oder C₁-C₁₈-Alkyl, wobei die Reste R¹ gleich oder verschieden sein können, wenn sie mehrfach auftreten;
- R², R³: unabhängig voneinander Wasserstoff;
C₁-C₁₈-Alkyl, dessen Kohlenstoffkette durch eine oder mehrere Gruppierungen -O-, -S-, -CO-, -SO- und/oder -SO₂- unterbrochen sein kann und das ein- oder mehrfach durch C₁-C₁₂-Alkoxy, C₁-C₆-Alkylthio, Hydroxy, Mercapto, Halogen, Cyano, Nitro und/oder -COOR¹ substituiert sein kann;
Aryl oder Hetaryl, an das jeweils weitere gesättigte oder ungesättigte 5- bis 7-gliedrige Ringe, deren Kohlenstoffgerüst durch eine oder mehrere Gruppierungen -O-, -S-, -CO- und/oder -SO₂- unterbrochen sein kann, anneliert sein können, wobei das gesamte Ringsystem ein- oder mehrfach durch C₁-C₁₂-Alkyl und/oder die vorstehenden, als Substituenten für Alkyl genannten Reste substituiert sein kann;
- Hal: Chlor, Brom oder Iod;
- n: 1, 2 oder 3;
- x: 2 bis 8.

Halogenierte Rylencarbonsäureimide, vor allem bromierte Perylen-3,4-dicarbonsäuremonoimide, Terrylen-3,4:11,12-tetracarbonsäurediimide und Quaterrylen-3,4:13,14-tetracarbonsäurediimide, sind wichtige Zwischenprodukte bei der Synthese von Fluoreszenzfarbstoffen, Infrarotabsorbern und Pigmentadditiven auf Rylenbasis. Sie werden dabei durch nucleophile Substitutionsreaktionen (Austausch der Bromatome z.B. durch Phenoxyreste) und/oder Kondensationsreaktionen in der peri-Position (Bildung von C-C-Verknüpfungen unter Bromwasserstoffabspaltung) in die gewünschten Zielverbindungen überführt.

Zur Herstellung der bromierten Rylencarbonsäureimide ist bislang die Umsetzung der im Rylenkern unsubstituierten Rylencarbonsäureimide mit elementarem Brom in Abwesenheit von Wasser in chlorierten Kohlenwasserstoffen bekannt. Dabei wird üblicherweise in hoher Verdünnung und mit hohem Bromüberschuß und/oder bei hoher Temperatur gearbeitet. Die großen Lösungsmittelmengen sind dabei erforderlich, um unerwünschte Nebenreaktionen (Bromierung von aliphatischen und aromatischen Resten an den Imidstickstoffatomen sowie unkontrollierte Bromierung im Rylenkem) zu unterdrücken und die gewünschten Bromierungsprodukte selektiv zu erhalten.

So ist in der WO-A-96/22332 die Herstellung von bromierten Perylen-3,4-dicarbonsäuremonoimiden und Quaterrylen-3,4:13,14,tetracarbonsäurediimiden beschrieben. Explizit offenbart ist die Tri- bzw. Hexabromierung von N-(2,6-Diisopropylphenyl)-perylen-3,4-dicarbonsäuremonoimid und N,N'-Bis(2,6-diisopropylphenyl)quaterrylen-3,4:13,14-tetracarbonsäurediimid in 125 ml Chloroform/g Imid bei einem Molverhältnis von Brom zu Imid von 58,4 : 1 bei 61 °C.

Die WO-A-03/104232 betrifft die Bromierung von Terrylen-3,4:11,12-tetracarbonsäurediimiden. Explizit offenbart ist die Tetra- bzw. Dibromierung verschiedener Terrylen-3,4:11,12-tetracarbonsäurediimide in Lösungsmittelmengen von 70 bis 100 ml/g Imid bei einem Molverhältnis von Brom zu Imid von 5 bzw. 2,5 : 1 und Temperaturen von 65 bis 80°C bzw. 60 bis 65°C.

In Tetrahedron 59, S. 1191-1207 (2003) wird zwar die Herstellung von N-(2,5-Di-tert.-butylphenyl)tribromperylen-3,4-dicarbonsäuremonoimid durch Bromierung in 30 ml Chloroform/g Imid bei einem Molverhältnis von Brom zu Imid von 15,7 : 1 beschrieben, unter diesen Bedingungen kann jedoch keine selektive Bromierung erfolgen. Dementsprechend wird hier nach Säulenchromatographie nur eine Ausbeute von 32% erzielt.

Zur Herstellung von halogenierten Perylen-3,4:9,10-tetracarbonsäurediimiden werden üblicherweise alternative Reaktionswege beschritten. So können die Dibrom- bzw. die Tetrachlorimide durch Umsetzung von Perylen-3,4:9,10-tetracarbonsäuredianhydrid mit elementarem Brom bzw. Chlor in 100 gew.%iger Schwefelsäure und anschließende Imidierung erhalten werden (WO-A-97/22607 bzw. DE-A-25 19 790 und EP-A-227 980). Schließlich wird in der DE-A-34 34 049 auch die Chlorierung der Imide mit Sulfurylchlorid in Nitrobenzol beschrieben.

Der Erfindung lag die Aufgabe zugrunde, höherhalogenierte (d.h. mindestens drei Halogenatome im Molekül enthaltende) Rylencarbonsäureimide auf verfahrenstechnisch vorteilhafte Weise mit möglichst hoher Selektivität zugänglich zu machen und dabei insbesondere auch die für die Halogenierung benötigte Lösungsmittelmenge zu reduzieren.

Demgemäß wurde ein Verfahren zur Herstellung von halogenierten Rylencarbonsäureimiden der allgemeinen Formel I 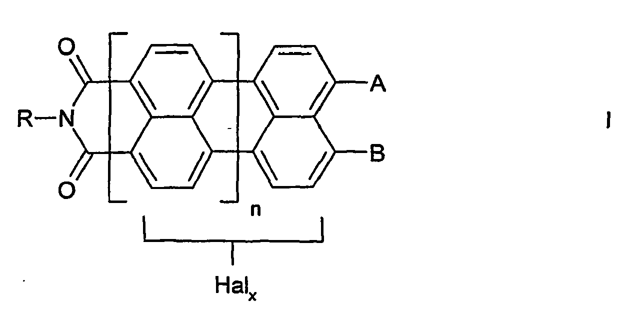 in der die Variablen folgende Bedeutung haben:
- A, B: gemeinsam ein Imidrest der Formel 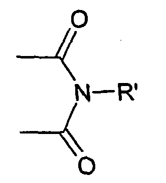 oder für den Fall, daß n gleich 1 ist, A und B auch beide Hal oder ein Rest Hal und der andere Rest Wasserstoff;
- R, R': unabhängig voneinander:
Wasserstoff;
(1) C₁-C₃₀-Alkyl, dessen Kohlenstoffkette durch eine oder mehrere Gruppierungen -O-, -S-, -NR¹-, -N=CR¹-, -C≡C-, -CR¹=CR¹-, -CO-, -SO- und/oder -SO₂- unterbrochen sein kann und das ein- oder mehrfach substituiert sein kann durch:
   (i) C₁-C₁₂-Alkoxy, C₁-C₆-Alkylthio, -C≡CR¹, -CR¹=CR¹₂, Hydroxy, Mercapto, Halogen, Cyano, Nitro, -NR²R³, -NR²COR³, -CONR²R³, -SO₂NR²R³, -COOR², -SO₃R², -PR²R³ und/oder -POR²R³;
   (ii) Aryl oder Hetaryl, an das weitere gesättigte oder ungesättigte 5- bis 7-gliedrige Ringe, deren Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann, anneliert sein können, wobei das gesamte Ringsystem ein- oder mehrfach substituiert sein kann durch: C₁-C₁₈-Alkyl, C₁-C₁₂-Alkoxy, C₁-C₆-Alkylthio, -C≡CR¹, -CR¹=CR¹₂, Hydroxy, Mercapto, Halogen, Cyano, Nitro, -NR²R³, -NR²COR³, -CONR²R³, -SO₂NR²R³, -COOR², -SO₃R², -PR²R³, -POR²R³, Aryl und/oder Hetaryl, das jeweils durch C₁-C₁₈-Alkyl, C₁-C₁₂-Alkoxy, Hydroxy, Mercapto, Halogen, Cyano, Nitro, -NR²R³, -NR²COR³, -CONR²R³, -SO₂NR²R³, -COOR², -SO₃R², -PR²R³ und/oder -POR²R³ substituiert sein kann;
   (iii) C₃-C₈-Cycloalkyl, dessen Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann und an das weitere gesättigte oder ungesättigte 5- bis 7-gliedrige Ringe, deren Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann, anneliert sein können, wobei das gesamte Ringsystem ein- oder mehrfach substituiert sein kann durch: C₁-C₁₈-Alkyl, C₁-C₁₂-Alkoxy, C₁-C₆-Alkylthio, -C≡CR¹, -CR¹=CR¹₂, Hydroxy, Mercapto, Halogen, Cyano, Nitro, -NR²R³, -NR²COR³, -CONR²R³, -SO₂NR²R³, -COOR², -SO₃R², -PR²R³ und/oder -POR²R³;
   (iv) einen Rest -U-Aryl, der ein- oder mehrfach durch die vorstehenden, als Substituenten für die Arylreste (ii) genannten Reste substituiert sein kann, wobei U eine Gruppierung -O-, -S-, -NR¹-, -CO-, -SO- oder -SO₂- bedeutet;
(2) C₃-C₈-Cycloalkyl, an das weitere gesättigte oder ungesättigte 5- bis 7-gliedrige Ringe, deren Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann, anneliert sein können, wobei das gesamte Ringsystem ein- oder mehrfach sein kann durch: die Reste (i), (ii), (iii), (iv) und/oder (v) C₁-C₃₀-Alkyl, dessen Kohlenstoffkette durch eine oder mehrere Gruppierungen -O-, -S-, -NR¹-, -N=CR¹-, -C≡C-, -CR¹=CR¹-, -CO-, -SO- und/oder -SO₂- unterbrochen sein kann und das ein- oder mehrfach substituiert sein kann durch: C₁-C₁₂-Alkoxy, C₁-C₆-Alkylthio, -C≡CR¹, -CR¹=CR¹₂, Hydroxy, Mercapto, Halogen, Cyano, Nitro, -NR²R³, -NR²COR³, -CONR²R³, -SO₂NR²R³, -COOR², -SO₃R², -PR²R³, -POR²R³, Aryl und/oder gesättigtes oder ungesättigtes C₄-C₇-Cycloalkyl, dessen Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann, wobei die Aryl- und Cycloalkylreste jeweils ein- oder mehrfach durch C₁-C₁₈-Alkyl und/oder die vorstehenden, als Substituenten für Alkyl genannten Reste substituiert sein können;
(3) Aryl oder Hetaryl, an das weitere gesättigte oder ungesättigte 5- bis 7-gliedrige Ringe, deren Kohlenstoffgerüst durch eine oder mehrere Gruppierungen -O-, -S-, -NR¹-, -N=CR¹-, -C≡C-, -CR¹=CR¹-, -CO-, -SO- und/oder -SO₂- unterbrochen sein kann, anneliert sein können, wobei das gesamte Ringsystem substituiert sein kann durch die Reste (i), (ii), (iii), (iv), (v) und/oder Aryl- und/oder Hetarylazo, das jeweils durch C₁-C₁₀-Alkyl, C₁-C₆-Alkoxy und/oder Cyano substituiert sein kann;
- R¹: Wasserstoff oder C₁-C₁₈-Alkyl, wobei die Reste R¹ gleich oder verschieden sein können, wenn sie mehrfach auftreten;
- R², R³: unabhängig voneinander Wasserstoff;
C₁-C₁₈-Alkyl, dessen Kohlenstoffkette durch eine oder mehrere Gruppierungen -O-, -S-, -CO-, -SO- und/oder -SO₂- unterbrochen sein kann und das ein- oder mehrfach durch C₁-C₁₂Alkoxy, C₁-C₆-Alkylthio, Hydroxy, Mercapto, Halogen, Cyano, Nitro und/oder -COOR¹ substituiert sein kann;
Aryl oder Hetaryl, an das jeweils weitere gesättigte oder ungesättigte 5- bis 7-gliedrige Ringe, deren Kohlenstoffgerüst durch eine oder mehrere Gruppierungen -O-, -S-, -CO- und/oder -SO₂- unterbrochen sein kann, anneliert sein können, wobei das gesamte Ringsystem ein- oder mehrfach durch C₁-C₁₂-Alkyl und/oder die vorstehenden, als Substituenten für Alkyl genannten Reste substituiert sein kann;
- Hal: Chlor, Brom oder Iod;
- n: 1, 2 oder 3;
- x: 2 bis 8,
durch Umsetzung eines Rylencarbonsäureimids der allgemeinen Formel II 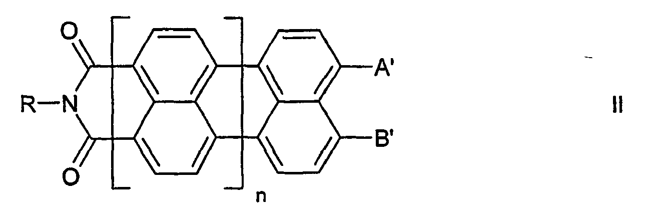 in der die Reste A' und B' gemeinsam einen Imidrest der Formel 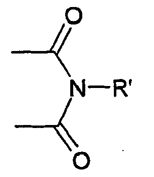 oder für den Fall, daß n gleich 1 ist, auch beide Wasserstoff bedeuten,
mit elementarem Halogen in Gegenwart eines inerten organischen Lösungsmittels L1 gefunden, welches dadurch gekennzeichnet ist, daß man dem Lösungsmittel L1 den sich bei der Umsetzung bildenden Halogenwasserstoff kontinuierlich entzieht.

Bevorzugte Anwendung findet das erfindungsgemäße Verfahren zur Herstellung von halogenierten Rylencarbonsäureimiden der Formel I, in der die Variablen folgende Bedeutung haben:
- R, R': unabhängig voneinander Wasserstoff;
C₁-C₃₀-Alkyl, dessen Kohlenstoffkette durch eine oder mehrere Gruppierungen -O-, -S-, -NR¹-, -CO- und/oder -SO₂- unterbrochen sein kann und das durch Cyano, C₁-C₆-Alkoxy, Aryl, das durch C₁-C₁₈-Alkyl oder C₁-C₆-Alkoxy substituiert sein kann, und/oder einen über ein Stickstoffatom ge-bundenen 5- bis 7-gliedrigen heterocyclischen Rest, der weitere Heteroatome enthalten und aromatisch sein kann, ein- oder mehrfach substituiert sein kann;
C₅-C₈-Cycloalkyl, dessen Kohlenstoffgerüst durch eine oder mehrere Gruppierungen -O-, -S- und/oder -NR¹- unterbrochen und/oder das durch C₁-C₆-Alkyl ein- oder mehrfach substituiert sein kann;
Aryl oder Hetaryl, das durch C₁-C₁₈-Alkyl, C₁-C₆-Alkoxy, Cyano, Halogen, -CONHR², -SO₂R², -SO₂NR²₂ und/oder Aryl- oder Hetarylazo, das jeweils durch C₁-C₁₀-Alkyl, C₁-C₆-Alkoxy oder Cyano substituiert sein kann, ein- oder mehrfach substituiert sein kann;
- R¹: Wasserstoff oder C₁-C₆-Alkyl;
- R²: Wasserstoff; C₁-C₁₈-Alkyl; Aryl oder Hetaryl, das jeweils durch C₁-C₆-Alkyl, C₁-C₆-Alkoxy, Halogen, Hydroxy, Carboxy oder Cyano substituiert sein kann.

Durch die Entfernung des Halogenwasserstoffs aus dem System wird die Selektivität der Halogenierung erhöht. Es nicht länger erforderlich, in hoher Verdünnung zu arbeiten, so daß sich die Umsetzung vor allem im großtechnischen Maßstab vereinfacht, da die Handhabung großer Lösungsmittelmengen entfällt.

Vorzugsweise entzieht man den Halogenwasserstoff beim erfindungsgemäßen Verfahren dadurch, daß man das Lösungsmittel L1 mit einem Lösungsmittel L2 in Kontakt bringt, von dem der gebildete Halogenwasserstoff aus dem Lösungsmittel L1 extrahiert wird und in dem sich der Halogenwasserstoff besser löst.

Das Lösungsmittel L2 sollte sich außerdem nicht mit dem Lösungsmittel L1 mischen (zwei Phasen bilden) oder einen höheren Siedepunkt als das Lösungsmittel L1 aufweisen und destillativ von diesem trennbar sein.

Zur Aufnahme des gebildeten Halogenwasserstoffs eignet sich insbesondere Wasser, das auch aufgrund der Nichtmischbarkeit mit den organischen Lösungsmitteln, die sich als Reaktionsmedium für Halogenierungen eignen, und seines im Vergleich zu diesen Lösungsmitteln hohen Siedepunkts das bevorzugte Lösungsmittel L2 ist.

Um die Wirksamkeit der Entfernung des Halogenwasserstoffs aus dem System weiter zu erhöhen, können erforderlichenfalls anstelle von reinem Wasser auch wäßrige Lösungen von Oxidationsmitteln und/oder Basen eingesetzt werden, die den Halogenwasserstoff zu Halogen oxidieren, das dann erneut zur Halogenierung zur Verfügung steht, bzw. den Halogenwasserstoff durch Säure-Base-Reaktion binden.

Als Oxidationsmittel eignen sich insbesondere oxidierend wirkende anorganische Salze, wie Peroxodisulfate, Nitrate, Bromate, Hypochlorite, Chlorate, Perchlorate und Permanganate.

Als Basen sind vor allem anorganische Basen, wie wasserlösliche Alkali- und Erdalkalimetallhydroxide, -carbonate und -hydrogencarbonate, insbesondere Lithium-, Natrium-, Kalium- und Magnesiumhydroxid, -carbonat und -hydrogencarbonat, geeignet.

Als inertes organisches Lösungsmittel L1 eignen sich für das erfindungsgemäße Verfahren solche Verbindungen, die unter den Reaktionsbedingungen gegenüber den Halogenen inert sind.

Unter diesen Lösungsmitteln sind solche bevorzugt, die mit der wäßrigen Phase L2 nicht mischbar sind (zwei Phasen bilden) oder einen niedrigeren Siedepunkt als Wasser aufweisen und von diesem destillativ trennbar sind.

Besonders geeignete Lösungsmittel L1 sind neben nitrierten aromatischen Kohlenwasserstoffen vor allem halogenierte aliphatische oder aromatische Kohlenwasserstoffe. Selbstverständlich können auch Mischungen dieser Lösungsmittel eingesetzt werden.

Beispiele für besonders geeignete halogenierte aliphatische Kohlenwasserstoffe L1 sind mehrfach chlorierte oder bromierte Kohlenwasserstoffe mit ein bis fünf C-Atomen, vor allem ein bis drei C-Atomen, wie Dichlormethan (Methylenchlorid), Dibrommethan, Trichlormethan (Chloroform), Tribrom-, Tetrachlor- und Tetrabrommethan, 1,2-Dichlor-, 1,2-Dibrom-, 1,1,1-Trichlor-, 1,1,2-Trichlor-, 1,1,1-Tribrom-, 1,1,2-Tribrom-, 1,1,1,2-Tetrachlor-, 1,1,2,2-Tetrachlor-, 1,1,1,2-Tetrabrom- und 1,1,2,2-Tetrabromethan und 1,2,3- und 1,1,2-Trichlorpropan..

Beispiele für besonders geeignete halogenierte aromatische Kohlenwasserstoffe L1 sind chloriertes und bromiertes Benzol, Alkylbenzol und Naphthalin, wie Chlor- und Brombenzol, die isomeren Dichlor-, Dibrom- und Trichlorbenzole, die isomeren Chlor- und Bromtoluole und 1-Chlor-, 2-Chlor- und 1-Bromnaphthalin.

Beispiele für besonders geeignete nitrierte aromatische Kohlenwasserstoffe L1 sind nitriertes Benzol und Alkylbenzol, wie Nitrobenzol und 2- und 4-Nitrotoluol.

Bevorzugte Lösungsmittel L1 sind Chloroform, Methylenchlorid und Chlorbenzol, wobei Chloroform und Methylenchlorid besonders bevorzugt sind.

Einer der wesentlichen Vorteile des erfindungsgemäßen Verfahrens ist, daß man die Einsatzmenge des inerten organischen Lösungsmittels gegenüber der üblichen Vorgehensweise deutlich verringern kann. So kommen in der Regel 1 bis 50 ml, bevorzugt 2 bis 20 ml und besonders bevorzugt 5 bis 15 ml Lösungsmittel L1 je g zu halogenierendes Rylencarbonsäureimid II zum Einsatz.

Die Einsatzmenge des bei der bevorzugten Ausführungsform des erfindungsgemäßen Verfahrens eingesetzten Lösungsmittels L2 (Wasser) beträgt üblicherweise 0,2 bis 5 ml, bevorzugt 0,5 bis 2,5 ml und besonders bevorzugt 0,8 bis 2 ml je ml Lösungsmittel L1.

Mit dem erfindungsgemäßen Verfahren können die halogenierten Rylencarbonsäureimide der Formel I hergestellt werden, d.h. die Rylencarbonsäureimide der Formel II, also Perylen-3,4-dicarbonsäuremonoimide, Perylen-3,4:9,10-tetracarbonsäurediimide, Terrylen-3,4:11,12-tetracarbonsäurediimide und Quaterrylen-3,4:13,14-tetracarbonsäurediimide, können halogeniert werden.

Besondere Bedeutung hat das erfindungsgemäße Verfahren zur Halogenierung, bevorzugt zur Chlorierung und besonders bevorzugt zur Bromierung von Perylen-3,4-dicarbonsäuremonoimiden und vor allem von Terrylen-3,4:11,12-tetracarbonsäurediimiden und Quatenylen-3,4:13,14-tetracarbonsäuredümiden.

Mit Hilfe des erfindungsgemäßen Verfahrens können insbesondere die höherhalogenierten Rylencarbonsäureimide gezielt hergestellt werden. So können z.B. die Perylen-3,4-dicarbonsäuremonoimide bis zu fünffach, bevorzugt dreifach, die Terrylen-3,4:11,12-tetracarbonsäurediimide bis zu sechsfach, vorzugsweise vierfach, und die Quaterrylen-3,4:13,14-tetracarbonsäurediimide bis zu achtfach, bevorzugt vierfach und besonders bevorzugt sechsfach, bromiert werden.

Alle in den Formeln I und II auftretenden Alkylgruppen können geradkettig oder verzweigt sein. Wenn die Alkylgruppen substituiert sind, tragen sie in der Regel 1 oder 2 Substituenten.

Cycloalkylgruppen und aromatische Reste, die substituiert sind, können im allgemeinen bis zu 3, bevorzugt 1 oder 2, der genannten Substituenten aufweisen.

Als Beispiele für geeignete Reste R, R', R¹ und R² (bzw. für deren Substituenten) seien im einzelnen genannt:
Methyl, Ethyl, Propyl, Isopropyl, Butyl, Isobutyl, tert.-Butyl, Pentyl, Isopentyl, Neopentyl, tert.-Pentyl, Hexyl, 2-Methylpentyl, Heptyl, 1-Ethylpentyl, Octyl, 2-Ethylhexyl, Isooctyl, Nonyl, Isononyl, Decyl, Isodecyl, Undecyl, Dodecyl, Tridecyl, Isotridecyl, Tetradecyl, Pentadecyl, Hexadecyl, Heptadecyl, Octadecyl, Nonadecyl und Eicosyl (die obigen Bezeichnungen Isooctyl, Isononyl, Isodecyl und Isotridecyl sind Trivialbezeichnungen und stammen von den nach der Oxosynthese erhaltenen Alkoholen);
Methoxymethyl, 2-Methoxyethyl, 2-Ethoxyethyl, 2-Propoxyethyl, 2-Isopropoxyethyl, 2-Butoxyethyl, 2- und 3-Methoxypropyl, 2- und 3-Ethoxypropyl, 2- und 3-Propoxypropyl, 2- und 3-Butoxypropyl, 2- und 4-Methoxybutyl, 2- und 4-Ethoxybutyl, 2- und 4-Propoxybutyl, 3,6-Dioxaheptyl, 3,6-Dioxaoctyl, 4,8-Dioxanonyl, 3,7-Dioxaociyl, 3,7-Dioxanonyl, 4,7-Dioxaoctyl, 4,7-Dioxanonyl, 2- und 4-Butoxybutyl, 4,8-Dioxadecyl, 3,6,9-Trioxadecyl, 3,6,9-Trioxaundecyl, 3,6,9-Trioxadodecyl, 3,6,9,12-Tetraoxatridecyl und 3,6,9,12-Tetraoxatetradecyl;
Methylthiomethyl, 2-Methylthioethyl, 2-Ethylthioethyl, 2-Propylthioethyl, 2-Isopropylthioethyl, 2-Butylthioethyl, 2- und 3-Methylthiopropyl, 2- und 3-Ethylthiopropyl, 2- und 3-Propylthiopropyl, 2- und 3-Butylthiopropyl, 2- und 4-Methylthiobutyl, 2- und 4-Ethylthiobutyl, 2- und 4-Propylthiobutyl, 3,6-Dithiaheptyl, 3,6-Dithiaoctyl, 4,8-Dithianonyl, 3,7-Dithiaoctyl, 3,7-Dithianonyl, 2- und 4-Butylthiobutyl, 4,8-Dithiadecyl, 3,6,9-Trithiadecyl, 3,6,9-Trithiaundecyl, 3,6,9-Trithiadodecyl, 3,6,9,12-Tetrathiatridecyl und 3,6,9,12-Tetrathiatetradecyl;
2-Monomethyl- und 2-Monoethylaminoethyl, 2-Dimethylaminoethyl, 2- und 3- Dimethylaminopropyl, 3-Monoisopropylaminopropyl, 2- und 4-Monopropylaminobutyl, 2- und 4-Dimethylaminobutyl, 6-Methyl-3,6-diazaheptyl, 3,6-Dimethyl-3,6-diazaheptyl, 3,6-Diazaoctyl, 3,6-Dimethyl-3,6-diazaoctyl, 9-Methyl-3,6,9-triazadecyl, 3,6,9-Trimethyl-3,6,9-triazadecyl, 3,6,9-Triazaundecyl, 3,6,9-Trimethyl-3,6,9-triazaundecyl, 12-Methyl-3,6,9,12-tetraazatridecyl und 3,6,9,12-Tetramethyl-3,6,9,12-tetraazatridecyl;
(1-Ethylethyliden)aminoethylen, (1-Ethylethyliden)aminopropylen, (1-Ethylethyliden)-aminobutylen, (1-Ethylethyliden)aminodecylen und (1-Ethylethyliden)aminododecylen;
Propan-2-on-1-yl, Butan-3-on-1-yl, Butan-3-on-2-yl und 2-Ethylpentan-3-on-1-yl;
2-Methylsulfoxidoethyl, 2-Ethylsulfoxidoethyl, 2-Propylsulfoxidoethyl, 2-Isopropylsulfoxidoethyl, 2-Butylsulfoxidoethyl, 2- und 3-Methylsulfoxidopropyl, 2- und 3-Ethylsulfoxidopropyl, 2- und 3-Propylsulfoxidopropyl, 2- und 3-Butylsulfoxidopropyl, 2- und 4-Methylsulfoxidobutyl, 2- und 4-Ethylsulfoxidobutyl, 2- und 4-Propylsulfoxidobutyl und 4-Butylsulfoxidobutyl;
2-Methylsulfonylethyl, 2-Ethylsulfonylethyl, 2-Propylsulfonylethyl, 2-Isopropylsulfonylethyl, 2-Butylsulfonylethyl, 2- und 3-Methylsulfonylpropyl, 2- und 3-Ethylsulfonylpropyl, 2- und 3-Propylsulfonylpropyl, 2- und 3-Butylsulfonylpropyl, 2- und 4-Methylsulfonylbutyl, 2- und 4-Ethylsulfonylbutyl, 2- und 4-Propylsulfonylbutyl und 4-Butylsulfonylbutyl;
Carboxymethyl, 2-Carboxyethyl, 3-Carboxypropyl, 4-Carboxybutyl, 5-Carboxypentyl, 6-Carboxyhexyl, 8-Carboxyoctyl, 10-Carboxydecyl, 12-Carboxydodecyl und 14-Carboxytetradecyl;
Sulfomethyl, 2-Sulfoethyl, 3-Sulfopropyl, 4-Sulfobutyl, 5-Sulfopentyl, 6-Sulfohexyl, 8-Sulfooctyl, 10-Sulfodecyl, 12-Suffododecyl und 14-Sulfotetradecyl;
2-Hydroxyethyl, 2- und 3-Hydroxypropyl, 1-Hydroxyprop-2-yl, 3- und 4-Hydroxybutyl, 1-Hydroxybut-2-yl und 8-Hydroxy-4-oxaoctyl;
Cyanomethyl, 2-Cyanoethyl, 3-Cyanopropyl, 2-Methyl-3-ethyl-3-cyanopropyl, 7-Cyano-7-ethylheptyl und 4,7-Dimethyl-7-cyanoheptyl;
2-Chlorethyl, 2- und 3-Chlorpropyl, 2-, 3- und 4-Chlorbutyl, 2-Bromethyl, 2- und 3-Brompropyl und 2-, 3- und 4-Brombutyl;
2-Nitroethyl, 2- und 3-Nitropropyl und 2-, 3- und 4-Nitrobutyl;
Methoxy, Ethoxy, Propoxy, Isopropoxy, Butoxy, Isobutoxy, sec.-Butoxy, tert.-Butoxy, Pentoxy, Isopentoxy, Neopentoxy, tert.-Pentoxy und Hexoxy;
Methylthio, Ethylthio, Propylthio, Isopropylthio, Butylthio, Isobutylthio, sec.-Butylthio, tert.-Butylthio, Pentylthio, Isopentylthio, Neopentylthio, tert.-Pentylthio und Hexylthio;
Ethinyl, 1- und 2-Propinyl, 1-, 2- und 3-Butinyl, 1-, 2-, 3- und 4-Pentinyl, 1-, 2-, 3-, 4- und 5-Hexinyl, 1-, 2-, 3-, 4-, 5-, 6-, 7-, 8- und 9-Decinyl, 1-, 2-, 3-, 4-, 5-, 6-, 7-, 8-, 9-, 10- und 11-Dodecinyl und 1-, 2-, 3-, 4-, 5-, 6-, 7-, 8-, 9-, 10-, 11-, 12-, 13-, 14-, 15-, 16- und 17-Octadecinyl;
Ethenyl, 1- und 2-Propenyl, 1-, 2- und 3-Butenyl, 1-, 2-, 3- und 4-Pentenyl, 1-, 2-, 3-, 4- und 5-Hexenyl, 1-, 2-, 3-, 4-, 5-, 6-, 7-, 8- und 9-Decenyl, 1-, 2-, 3-, 4-, 5-, 6-, 7-, 8-, 9-, 10- und 11-Dodecenyl und 1-, 2-, 3-, 4-, 5-, 6-, 7-, 8-, 9-, 10-, 11-, 12-, 13-, 14-, 15-, 16- und 17-Octadecenyl;
Methylamino, Ethylamino, Propylamino, Isopryplamino, Butylamino, Isobutylamino, Pentylamino, Hexylamino, Dimethylamino, Methylethylamino, Diethylamino, Dipropylamino, Diisopropylamino, Dibutylamino, Diisobutylamino, Dipentylamino, Dihexylamino, Dicyclopentylamino, Dicyclohexylamino, Dicycloheptylamino, Diphenylamino und Dibenzylamino;
Formylamino, Acetylamino, Propionylamino und Benzoylamino;
Carbamoyl, Methylaminocarbonyl, Ethylaminocarbonyl, Propylaminocarbonyl, Butylaminocarbonyl, Pentylaminocarbonyl, Hexylaminocarbonyl, Heptylaminocarbonyl, Octylaminocarbonyl, Nonylaminocarbonyl, Decylaminocarbonyl und Phenylaminocarbonyl;
Methoxycarbonyl, Ethoxycarbonyl, Propoxycarbonyl, Isopropoxycarbonyl, Hexoxycarbonyl, Dodecyloxycarbonyl, Octadecyloxycarbonyl, Phenoxycarbonyl, (4-tert.-Butylphenoxy)carbonyl und (4-Chlorphenoxy)carbonyl;
Methoxysulfonyl, Ethoxysulfonyl, Propoxysulfonyl, Isopropoxysulfonyl, Butoxysulfonyl, Isobutoxysulfonyl, tert.-Butoxysulfonyl, Hexoxysulfonyl, Dodecyloxysulfonyl, Octadecyloxysulfonyl, Phenoxysulfonyl, 1- und 2-Naphthyloxysulfonyl, (4-tert.-Butylphenoxy)-sulfonyl und (4-Chlorphenoxy)sulfonyl;
Diphenylphosphino, Di-(o-tolyl)phosphino und Diphenylphosphinoxido;
Chlor, Brom und Iod;
Methylsulfonyl, Ethylsulfonyl, Propylsulfonyl, Isopropylsulfonyl, Butylsulfonyl, Isobutylsulfonyl, sec.-Butylsulfonyl, tert.-Butylsufonyl, Pentylsulfonyl, Isopentylsulfonyl, Neopentylsulfonyl, tert.-Pentylsulfonyl, Hexylsulfonyl, Heptylsulfonyl, Octylsulfonyl, 2-Ethylhexylsulfonyl, Nonylsulfonyl, Decylsulfonyl, 3-Propylheptylsulfonyl, Dodecylsulfonyl und Octydecylsulfonyl;
Aminosulfonyl, N,N-Dimethylaminosulfonyl, N,N-Diethylaminosulfonyl, N,N-Dipropylaminosulfonyl, N,N-Diisopropylaminosulfonyl, N,N-Dibutylaminosulfonyl, N,N-Diisobutylaminosulfonyl, N,N-Di-sec.-butylaminosulfonyl, N,N-Di-tert.-butylaminosulfonyl, N,N-Dipentylaminosulfonyl, N,N-Dihexylaminosulfonyl, N,N-Diheptylaminosulfonyl, N,N-Dioctylaminosulfonyl, N,N-Dinonylaminosulfonyl, N,N-Didecylaminosulfonyl, N,N-Didodecylaminosulfonyl, N-Methyl-N-ethylaminosulfonyl, N-Methyl-N-dodecylaminosulfonyl, N-Dodecylaminosulfonyl, (N,N-Dimethylamino)ethylaminosulfonyl, N,N-(Propoxyethyl)dodecylaminosulfonyl, N,N-Diphenylaminosulfonyl, N,N-(4-tert.-Butylphenyl)octadecylaminosulfonyl und N,N-Bis(4-Chlorphenyl)aminosulfonyl;
Phenylazo, 2-Naphthylazo, 2-Pyridylazo und 2-Pyrimidylazo;
Phenyl, 1- und 2-Naphthyl, 2- und 3-Pyrryl, 2-, 3- und 4-Pyridyl, 2-, 4- und 5-Pyrimidyl, 3-, 4- und 5-Pyrazolyl, 2-, 4- und 5-Imidazolyl, 2-, 4- und 5-Thiazolyl, 3-(1,2,4-Triazyl), 2-(1,3,5-Triazyl), 6-Chinaldyl, 3-, 5-, 6- und 8-Chinolinyl, 2-Benzoxazolyl, 2-Benzothiazolyl, 5-Benzothiadiazolyl, 2- und 5-Benzimidazolyl und 1- und 5- Isochinolyl;
2-, 3- und 4-Methylphenyl, 2,3-, 2,4-, 2,5-, 3,5- und 2,6-Dimethylphenyl, 2,4,6-Trimethylphenyl, 2-, 3- und 4-Ethylphenyl, 2,3-, 2,4-, 2,5-, 3,5- und 2,6-Diethylphenyl, 2,4,6-Triethylphenyl, 2-, 3- und 4-Propylphenyl, 2,3-, 2,4-, 2,5-, 3,5- und 2,6-Dipropylphenyl, 2,4,6-Tripropylphenyl, 2-, 3- und 4-Isopropylphenyl, 2,3-, 2,4-, 2,5-, 3,5- und 2,6-Diisopropylphenyl, 2,4,6-Triisopropylphenyl, 2-, 3- und 4-Butylphenyl, 2,3-, 2,4-, 2,5-, 3,5- und 2,6-Dibutylphenyl, 2,4,6-Tributylphenyl, 2-, 3- und 4-Isobutylphenyl, 2,3-, 2,4-, 2,5-, 3,5- und 2,6-Diisobutylphenyl, 2,4,6-Triisobutylphenyl, 2-, 3- und 4-sec.-Butylphenyl, 2,3-, 2,4-, 2,5-, 3,5- und 2,6-Di-sec.-butylphenyl und 2,4,6-Tri-sec.-butylphenyl, 2-, 3- und 4-tert.-Butylphenyl, 2,3-, 2,4-, 2,5-, 3,5- und 2,6-Di-tert.-butylphenyl, 2,4,6-Tri-tert.-butylphenyl; 2-, 3- und 4-Methoxyphenyl, 2,3-, 2,4-, 2,5-, 3,5- und 2,6-Dimethoxyphenyl, 2,4,6-Trimethoxyphenyl, 2-, 3- und 4-Ethoxyphenyl, 2,3-, 2,4-, 2,5-, 3,5- und 2,6-Diethoxyphenyl, 2,4,6-Triethoxyphenyl, 2-, 3- und 4-Propoxyphenyl, 2,3-, 2,4-, 2,5-, 3,5- und 2,6-Dipropoxyphenyl, 2-, 3- und 4-Isopropoxyphenyl, 2,3-, 2,4-, 2,5-, 3,5- und 2,6-Diisopropoxyphenyl und 2-, 3- und 4-Butoxyphenyl; 2-, 3- und 4-Chlorphenyl, und 2,3-, 2,4-, 2,5-, 3,5- und 2,6-Dichlorphenyl; 2-, 3- und 4-Hydroxyphenyl und 2,3-, 2,4-, 2,5-, 3,5- und 2,6-Dihydroxyphenyl; 2-, 3- und 4-Cyanophenyl; 3- und 4-Carboxyphenyl; 3- und 4-Carboxamidophenyl, 3- und 4-N-Methylcarboxamidophenyl und 3- und 4-N-Ethylcarboxamidophenyl; 3- und 4-Acetylaminophenyl, 3- und 4-Propionylaminophenyl und 3- und 4-Butyrylaminophenyl; 3- und 4-N-Phenylaminophenyl, 3- und 4-N-(o-Tolyl)aminophenyl, 3- und 4-N-(m-Tolyl)aminophenyl und 3- und 4-N-(p-Tolyl)aminophenyl; 3- und 4-(2-Pyridyl)aminophenyl, 3- und 4-(3-Pyridyl)Aminophenyl, 3- und 4-(4-Pyridyl)aminophenyl, 3- und 4-(2-Pyrimidyl)aminophenyl und 4-(4-Pyrimidyl)-aminophenyl; 3- und 4-Methylsulfonylphenyl, 3- und 4-Ethylsulfonylphenyl, 3- und 4-Propylsulfonylphenyl, 3- und 4-Isopropylsulfonylphenyl, 3- und 4-Butylsulfonylphenyl, 3- und 4-Isobutylsulfonylphenyl, 3- und 4-(sec.-Butylsulfonyl)phenyl, 3- und 4-(tert.-Butylsufonyl)phenyl, 3- und 4-Pentylsulfonylphenyl, 3- und 4-Isopentylsulfonylphenyl, 3- und 4-Neopentylsulfonylphenyl, 3- und 4-(tert.-Pentylsulfonyl)phenyl, 3- und 4-Hexylsulfonylphenyl, 3- und 4-Heptylsulfonylphenyl, 3- und 4-Octylsulfonylphenyl, 3- und 4-(2-Ethylhexyl)sulfonylphenyl, 3- und 4-Nonylsulfonylphenyl, 3- und 4-Decylsulfonylphenyl, 3- und 4-(3-Propylheptyl)sulfonylphenyl, 3- und 4-Dodecylsulfonylphenyl und 3- und 4-Octadecylsulfonylphenyl; 3- und 4-Dimethylaminosulfonylphenyl, 3- und 4-Diethylaminosulfonylphenyl, 3- und 4-Methylethylaminosulfonylphenyl, 3- und 4-Dipropylaminosulfonylphenyl, 3- und 4-Diisopropylaminosulfonylphenyl, 3- und 4-Dibutylaminosulfonylphenyl, 3- und 4-Diisobutylaminosulfonylphenyl, 3- und 4-Di-sec.-butylaminosulfonylphenyl, 3- und 4-Di-tert.-butylaminosulfonylphenyl, 3- und 4-Dipentylaminosulfonylphenyl, 3- und 4-Dihexylaminosulfonylphenyl, 3- und 4-Diheptylaminosulfonylphenyl, 3- und 4-Dioctylaminosulfonylphenyl, 3- und 4-Dinonylaminosulfonylphenyl, 3- und 4-Didecylaminosulfonylphenyl und 3- und 4-Didodecylaminosulfonylphenyh
4-Phenylazophenyl, 4-(1-Naphthylazo)phenyl, 4-(2-Naphthylazo)phenyl, 4-(4-Naphthylazo)phenyl, 4-(2-Pyridylazo)phenyl, 4-(3-Pyridylazo)phenyl, 4-(4-Pyridylazo)phenyl, 4-(2-Pyrimidylazo)phenyl, 4-(4-Pyrimidylazo)phenyl und 4-(5-Pyrimidylazo)phenyl;
Cyclopropyl, Cyclobutyl, Cyclopentyl, 2- und 3-Methylcyclopentyl, 2- und 3-Ethylcyclopentyl, Cyclohexyl, 2-, 3- und 4-Methylcyclohexyl, 2-, 3- und 4-Ethylcyclohexyl, 3- und 4-Propylcyclohexyl, 3- und 4-Isopropylcyclohexyl, 3- und 4-Butylcyclohexyl, 3- und 4-sec.-Butylcyclohexyl, 3- und 4-tert.-Butylcyclohexyl, Cycloheptyl, 2-, 3- und 4-Methylcycloheptyl, 2-, 3- und 4-Ethylcycloheptyl, 3- und 4-Propylcycloheptyl, 3- und 4-Isopropylcycloheptyl, 3- und 4-Butylcycloheptyl, 3- und 4-sec.-Butylcycloheptyl, 3- und 4-tert.-Butylcycloheptyl, Cyclooctyl, 2-, 3-, 4- und 5-Methylcyclooctyl, 2-, 3-, 4- und 5-Ethylcyclooctyl, 3-, 4- und 5-Propylcyclooctyl, 3- und 4-Hydroxycyclohexyl, 3- und 4-Nitrocyclohexyl und 3- und 4-Chlorcyclohexyl;
1-, 2- und 3-Cyclopentenyl, 1-, 2-, 3- und 4-Cyclohexenyl, 1-, 2- und 3-Cycloheptenyl und 1-, 2-, 3- und 4-Cyclooctenyl;
2-Dioxanyl, 1-Morpholinyl, 1-Thiomorpholinyl, 2- und 3-Tetrahydrofuryl, 1-, 2- und 3-Pyrrolidinyl, 1-Piperazyl, 1-Diketopiperazyl und 1-, 2-, 3- und 4-Piperidyl;
Dipropylaminosulfonyl, Diisopropylaminosulfonyl, Dibutylaminosulfonyl, Diisobutylaminosulfonyl, Di-sec.-butylaminosulfonyl, Di-tert.-butylaminosulfonyl, Dipentylaminosulfonyl, Dihexyl-aminosulfonyl, Diheptylaminosulfonyl, Dioctylaminosulfonyl, Dinonylaminosulfonyl, Didecylaminosulfonyl und Didodecylaminosulfonyl.

Die Halogenierung erfolgt durch Umsetzung mit dem elementarem Halogen, worunter erfindungsgemäß neben elementarem Chlor, Brom und Iod auch Interhalogene wie Iodchlorid verstanden werden sollen.

Als Halogen sind Chlor und Brom bevorzugt, wobei Brom besonders bevorzugt ist.

Je mol zu halogenierendes Rylencarbonsäureimid II werden in der Regel 3 bis 50 mol, vorzugsweise 5 bis 30 mol Halogen eingesetzt.

Gewünschtenfalls kann das Halogen portionsweise während der Reaktion zugesetzt werden. Halogenverluste können durch Arbeiten im geschlossenen System (Autoklav) vermieden werden.

Zur Katalysierung der Chlorierung und Bromierung kann Iod zugesetzt werden, in den meisten Fällen ist dies jedoch nicht erforderlich. Wird Iod zugesetzt, so liegen die Mengen zweckmäßig bei 0,1 bis 10 mol-%, bezogen auf das Halogen.

Die Reaktionstemperatur beträgt in der bei Regel 5 bis 85°C, bevorzugt 20 bis 65°C.

Verfahrenstechnisch kann man beim erfindungsgemäßen Verfahren nach verschiedenen Varianten vorgehen.

Bei einer bevorzugten Ausführungsform führt man die Halogenierung unter Rühren in einem Zweiphasengemisch aus organischem Lösungsmittel L1 und wäßriger Phase L2 durch.

Die Lösungsmittelphase L1, die das Halogen weitestgehend enthält und in der das zu bromierende Rylencarbonsäureimid II gelöst, benetzt oder suspendiert ist, ist hier permanent mit der wäßrigen Phase L2 in Kontakt, so daß der während der Reaktion entstehende Halogenwasserstoff kontinuierlich aus der Lösungsmittelphase L1 ausgewaschen wird.

Gewünschtenfalls kann man die wäßrige Phase L2 während der Reaktion abtrennen und durch eine frische wäßrige Phase L2 austauschen.

Bei dieser Ausführungsform unterliegt der Siedepunkt des organischen Lösungsmittels L1 keiner Einschränkung. Es muß lediglich gewährleistet sein, daß sich Lösungsmittel L1 und L2 nicht mischen, es also zur Ausbildung zweier Phasen kommt.

Diese erste Ausführungsform des erfindungsgemäßen Verfahrens ist insbesondere für die Halogenierung der Perylen-3,4-dicarbonsäuremonoimide und Terrylen-3,4:11,12-tetracarbonsäurediimide geeignet.

Bei einer weiteren bevorzugten Ausführungsform des erfindungsgemäßen Verfahrens nimmt man die Halogenierung in dem Lösungsmittel L1 vor und bringt dieses wiederholt mit dem Lösungsmittel L2 in Kontakt und entzieht auf diese Weise den gebildeten Halogenwasserstoff.

Diese Ausführungsform basiert auf der besseren Löslichkeit der halogenierten Imide und eignet sich daher bevorzugt zur Halogenierung der Quaterrylen-3,4:13,14-teracarbonsäurediimide.

Die Halogenierung erfolgt hier am im Lösungsmittel L1 suspendierten Rylencarbonsäureimid II, wobei Rührung nicht erforderlich ist. Das im Lösungsmittel L1 gelöste halogenierte Rylencarbonsäureimid I wird wiederholt durch Filtration vom noch nicht umgesetzten Rylencarbonsäureimid II abgetrennt. Das organische Filtrat (Lösungsmittel L1, Brom, halogeniertes Produkt und Halogenwasserstoff) wird dann zur Entfernung des Halogenwasserstoffs mit wäßriger Phase L2 ausgewaschen, abgetrennt und wieder dem Rückstand zugeführt. Gewünschtenfalls kann man das Rylencarbonsäureimid II sowie das Brom portionsweise zusetzen.

Die Abtrennung der organischen Phase L1 vom Waschwasser L2 kann auf verschiedenen Weisen erfolgen.

Eine Abtrennungsvariante bildet die Trennung der flüssigen Phasen direkt nach der Filtration oder nach einer gewissen Nachrührzeit. Im letzteren Fall kann noch nicht vollständig halogeniertes Rylencarbonsäureimid weiter zum Zielprodukt halogenieren.

Die Abtrennung kann jedoch auch durch Abdestillieren von Lösungsmittel L1 und Brom erfolgen. Bei dieser Variante muß das Lösungsmittel L1 einen niedrigeren Siedepunkt als die wäßrige Phase L2 aufweisen und somit von Wasser destillativ trennbar sein.

Die Aufarbeitung des Reaktionsansatzes (der organischen Phase L1, die das Halogenierungsprodukt sowie Halogenreste enthält) kann so erfolgen, daß man zunächst das Halogen durch Ausblasen entfernt, dann das Lösungsmittel L1 weitgehend abdestilliert und das Halogenierungsprodukt abfiltriert.

Zur weiteren Reinigung kann das abfiltrierte Produkt mit Wasser und wäßrigen Lösungen von Reduktionsmitteln gewaschen werden. Durch das Reduktionsmittel werden verbliebene Halogenspuren zu Halogenid reduziert. Geeignete Reduktionsmittel sind z.B. Dithionite, Thiosulfate, Sulfite, Hydrogensulfite, Nitrite und Formiate. Teilweise (z.B, bei den Dithioniten und Thiosulfaten) kann die Verwendung alkalischer Lösungen zweckmäßig sein.

Es ist jedoch auch möglich, die organische, das Halogenierungsprodukt enthaltende Phase, gewünschtenfalls nach vorherigem Ausblasen z.B. mit Stickstoff, zur Entfernung von verbliebenen Halogenspuren mit wäßrigen Reduktionsmittellösungen zu behandeln und das Lösungsmittel anschließend abzudestillieren.

Mit Hilfe des erfindungsgemäßen Verfahrens können die halogenierten Rylencarbonsäureimide I auf verfahrenstechnisch einfache Weise in hoher Ausbeute (in der Regel über 90%) und hoher Reinheit (üblicherweise 75 bis 100%) hergestellt werden. Die erhaltenen Produkte zeichnen sich dadurch aus, daß sie überhalogenierte Anteile nur in Spuren (üblicherweise unter 5%) enthalten und eine Halogenierung außerhalb des Rylenkerns praktisch nicht auftritt. Unterhalogenierung kann selbstverständlich durch eine ausreichende Reaktionszeit weitgehend vermieden werden.

### Beispiele

### Beispiel 1

20,0 g (21 mmol) von N,N'-Bis(2,6-diisopropylphenyl)quaterrylen-3,4:13,14-tetra-carbonsäurediimid wurden in eine Glasfaserhülse für eine Soxhlett-Apparatur gefüllt. In den Destillationskolben der Soxhlett-Apparatur wurden 130 ml Chloroform, 105 ml Wasser und 18,9 ml (370 mmol) Brom gegeben. Dann wurde der Inhalt des Destillationskolbens zum Rückfluß erhitzt. Ein Gemisch aus Chloroform und Brom destillierte ab und benetzte den Feststoff in der Hülse. Nach dem Vollaufen der Hülse lief ein Gemisch von Chloroform, Brom und bromiertem Produkt in den Destillationskolben ab. Das Chloroform wurde durch das Wasser im Destillationskolben ausgewaschen und zusammen mit dem Brom erneut zum Destillieren gebracht. In 3 Portionen wurden weitere 11,4 ml (220 mmol) Brom zugegeben. Nach 70 stündiger Reaktionszeit wurden verbliebenes Brom und Chloroform abdestilliert.

Der im Destillationskolben befindliche Rückstand wurde abfiltriert, mit Wasser und 20 gew.%iger wäßriger Natriumsulfitlösung gewaschen und im Vakuum getrocknet.

Es wurden 24,6 g N,N'-Bis(2,6-diisopropylphenyl)hexabromquaterrylen-3,4:13,14-tetra-carbonsäurediimid (R_{f} (Toluol) = 0,17) erhalten (82% Ausbeute).

### Vergleichsbeispiel 1V

N,N'-Bis(2,6-diisopropylphenyl)quaterrylen-3,4:13,14-tetracarbonsäurediimid wurde analog in derselben Apparatur, jedoch in Abwesenheit von Wasser bromiert.

Neben dem gewünschten hexabromierten Produkt wurden deutliche Mengen überbromierter Produkte erhalten, die im Dünnschichtchromatogramm an der geringeren Polarität (höhere R_{f}-Werte) erkennbar sind: R_{f} (Toluol) = 0,17; 0,33; 0,61.

### Beispiel 2

19,1 g (0,023 mol) N,N'-Bis(2,6-diisopropylphenyl)terrylen-3,4:11,12-tetracarbonsäurediimid wurden in 250 ml Chloroform gelöst und mit 500 ml Wasser versetzt. Anschließend wurden 18,6 ml (0,36 mol) Brom langsam in 2 min zugegeben. Nach 5 stündigem Rühren bei 40°C und 15 stündigem Rühren bei Raumtemperatur wurden verbliebenes Brom und Chloroform abdestilliert.

Der Rückstand wurde abfiltriert, mit Wasser und 20 gew.%iger wäßriger Natriumsulfitlösung gewaschen und im Vakuum getrocknet.

Es wurden 26,1 g N,N'-Bis(2,6-diisopropylphenyl)tetrabromterrylen-3,4:11,12-tetra-carbonsäurediimid (R_{f} (Methylenchlorid) = 0,75; Bromgehalt: 27,6% (gef.); 27,8% (ber.)) erhalten (99% Ausbeute).

### Beispiel 3

5,0 g (10 mmol) N-(2,6-Diisopropylphenyl)perylen-3,4-dicarbonsäuremonoimid wurden in 65 ml Chloroform gelöst und mit 130 ml Wasser versetzt. Anschließend wurden 7 ml (130 mmol) Brom zugegeben und auf 30°C erhitzt. Nach 5 stündigem Rühren bei dieser Temperatur und weiterem 60 stündigen Rühren bei Raumtemperatur wurde verbliebenes Brom durch Ausblasen mit Stickstoff entfernt.

Organische und wäßrige Phase wurden getrennt. Dann wurde die organische Phase zweimal mit 20 gew.-%iger wäßriger alkalischer Natriumthiosulfatlösung ausgeschüttelt, mit Magnesiumsulfat getrocknet und eingeengt.

Es wurden 7,0 g N-(2,6-Diisopropylphenyl)tribromperylen-3,4-dicarbonsäureimid erhalten (98% Ausbeute). Dabei handelt es sich um zwei Isomere im Verhältnis von etwa 1 : 6: R_{f} (Toluol) = 0,25; 0,36.

### Vergleichsbeispiel 3V

10,0 g (20 mmol) N-(2,6-Diisopropylphenyl)perylen-3,4-dicarbonsäuremonoimid wurden in 400 ml Chloroform gelöst und nach Zugabe von 50 ml (975 mmol) Brom 6 h auf 70°C erhitzt. Die Aufarbeitung erfolgte analog Beispiel 3.

Neben dem gewünschten tribromierten Produkt wurden deutliche Mengen tetrabromierter Produkte erhalten, die im Dünnschichtchromatogramm an der geringeren Polarität (höhere R_{f}-Werte) erkennbar sind: R_{f} (Toluol) = 0,25; 0,36; 0,50; 0,58.

## Patentansprüche

1. Verfahren zur Herstellung von halogenierten Rylencarbonsäureimiden der allgemeinen Formel I 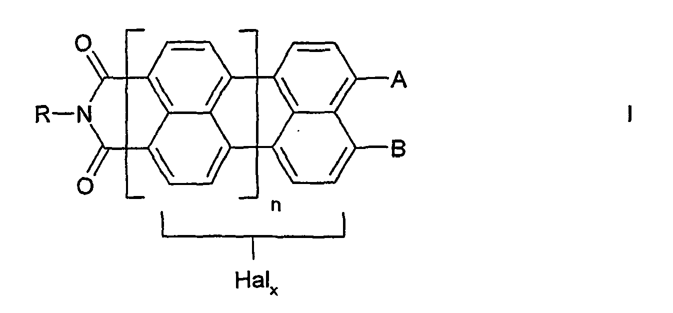 in der die Variablen folgende Bedeutung haben:
A, B gemeinsam ein Imidrest der Formel 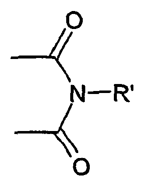 oder für den Fall, daß n gleich 1 ist, A und B auch beide Hal oder ein Rest Hal und der andere Rest Wasserstoff;
R, R' unabhängig voneinander:
Wasserstoff;
(1) C₁-C₃₀-Alkyl, dessen Kohlenstoffkette durch eine oder mehrere Grup-pierungen -O-, -S-, -NR¹-, -N=CR¹-, -C≡C-, -CR¹=CR¹-, -CO-, -SO- und/oder -SO₂- unterbrochen sein kann und das ein- oder mehrfach substituiert sein kann durch:
(i) C₁-C₁₂-Alkoxy, C₁-C₆-Alkylthio, -C≡CR¹, -CR¹=CR¹₂, Hydroxy, Mercapto, Halogen, Cyano, Nitro, -NR²R³, -NR²COR³, -CONR²R³, -SO₂NR²R³, -COOR², -SO₃R², -PR²R³ und/oder -POR²R³;
(ii) Aryl oder Hetaryl, an das weitere gesättigte oder ungesättigte 5- bis 7-gliedrige Ringe, deren Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann, anneliert sein können, wobei das gesamte Ringsystem ein- oder mehrfach substituiert sein kann durch: C₁-C₁₈-Alkyl, C₁-C₁₂-Alkoxy, C₁-C₆-Alkylthio, -C≡CR¹, -CR¹=CR¹₂, Hydroxy, Mercapto, Halogen, Cyano, Nitro, -NR²R³, -NR²COR³, -CONR²R³, -SO₂NR²R³, -COOR², -SO₃R ², -PR²R³, -POR²R³, Aryl und/oder Hetaryl, das jeweils durch C₁-C₁₈-Alkyl, C₁-C₁₂-Alkoxy, Hydroxy, Mercapto, Halogen, Cyano, Nitro, -NR²R³, -NR²COR³, -CONR²R³, -SO₂NR²R³, -COOR², -SO₃R², -PR²R³ und/oder -POR²R³ substituiert sein kann;
(iii) C₃-C₈-Cycloalkyl, dessen Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann und an das weitere gesättigte oder ungesättigte 5- bis 7-gliedrige Ringe, deren Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann, anneliert sein können, wobei das gesamte Ringsystem ein- oder mehrfach substituiert sein kann durch: C₁-C₁₈-Alkyl, C₁-C₁₂-Alkoxy, C₁-C₆-Alkylthio, -C≡CR¹, -CR¹=CR¹₂, Hydroxy, Mercapto, Halogen, Cyano, Nitro, -NR²R³, -NR²COR³, -CONR²R³, -SO₂NR²R³, -COOR², -SO₃R ², -PR²R³ und/oder -POR²R³;
(iv) einen Rest -U-Aryl, der ein- oder mehrfach durch die vorstehenden, als Substituenten für die Arylreste (ii) genannten Reste substituiert sein kann, wobei U eine Gruppierung -O-, -S-, -NR¹-, -CO-, -SO- oder -SO₂- bedeutet;
(2) C₃-C₈-Cycloalkyl, an das weitere gesättigte oder ungesättigte 5- bis 7-gliedrige Ringe, deren Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann, anneliert sein können, wobei das gesamte Ringsystem ein- oder mehrfach sein kann durch: die Reste (i), (ii), (iii), (iv) und/oder
(v) C₁-C₃₀-Alkyl, dessen Kohlenstoffkette durch eine oder mehrere Gruppierungen -O-, -S-, -NR¹-, -N=CR¹-, -C≡C-, -CR¹=CR¹-, -CO-, -SO- und/oder -SO₂- unterbrochen sein kann und das ein- oder mehrfach substituiert sein kann durch: C₁-C₁₂-Alkoxy, C₁-C₆-Alkylthio, -C≡CR¹, -CR¹=CR1₂, Hydroxy, Mercapto, Halogen, Cyano, Nitro, -NR²R³, -NR²COR³, -CONR²R³, -SO₂NR²R³, -COOR², -SO₃R², -PR²R³, -POR²R³, Aryl und/oder gesättigtes oder ungesättigtes C₄-C₇-Cycloalkyl, dessen Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann, wobei die Aryl- und Cycloalkylreste jeweils ein- oder mehrfach durch C₁-C₁₈-Alkyl und/oder die vorstehenden, als Substituenten für Alkyl genannten Reste substituiert sein können;
(3) Aryl oder Hetaryl, an das weitere gesättigte oder ungesättigte 5- bis 7-gliedrige Ringe, deren Kohlenstoffgerüst durch eine oder mehrere Gruppierungen -O-, -S-, -NR¹-, -N=CR¹-, -C≡C-, -CR¹=CR¹-, -CO-, -SO- und/oder -SO₂- unterbrochen sein kann, anneliert sein können, wobei das gesamte Ringsystem substituiert sein kann durch die Reste (i), (ii), (iii), (iv), (v) und/oder Aryl- und/oder Hetarylazo, das jeweils durch C₁-C₁₀-Alkyl, C₁-C₆-Alkoxy und/oder Cyano substituiert sein kann;
R¹ Wasserstoff oder C₁-C₁₈-Alkyl, wobei die Reste R¹ gleich oder verschieden sein können, wenn sie mehrfach auftreten;
R², R³ unabhängig voneinander Wasserstoff;
C₁-C₁₈-Alkyl, dessen Kohlenstoffkette durch eine oder mehrere Gruppierungen -O-, -S-, -CO-, -SO- und/oder -SO₂- unterbrochen sein kann und das ein- oder mehrfach durch C₁-C₁₂-Alkoxy, C₁-C₆-Alkylthio, Hydroxy, Mercapto, Halogen, Cyano, Nitro und/oder -COOR¹ substituiert sein kann;
Aryl oder Hetaryl, an das jeweils weitere gesättigte oder ungesättigte 5- bis 7-gliedrige Ringe, deren Kohlenstoffgerüst durch eine oder mehrere Gruppierungen -O-, -S-, -CO- und/oder -SO₂- unterbrochen sein kann, anneliert sein können, wobei das gesamte Ringsystem ein- oder mehrfach durch C₁-C₁₂-Alkyl und/oder die vorstehenden, als Substituenten für Alkyl genannten Reste substituiert sein kann;
Hal Chlor, Brom oder Iod;
n 1, 2 oder 3;
x 1 bis 8,
durch Umsetzung eines Rylencarbonsäureimids der allgemeinen Formel II 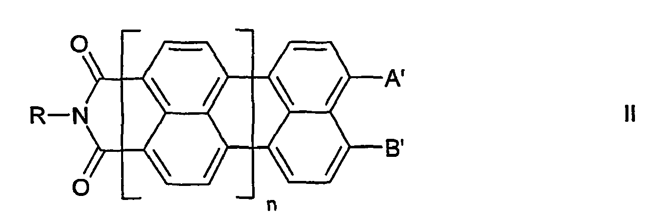 in der die Reste A' und B' gemeinsam einen Imidrest der Formel 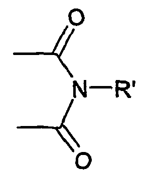 oder für den Fall, daß n gleich 1 ist, auch beide Wasserstoff bedeuten,
mit elementarem Halogen in Gegenwart eines inerten organischen Lösungsmittels L1, **dadurch gekennzeichnet, daß** man dem Lösungsmittel L1 den sich bei der Umsetzung bildenden Halogenwasserstoff kontinuierlich entzieht.

2. Verfahren anch Anspruch 1, **dadurch gekennzeichnet, daß** man es zur Herstellung von halogenierten Rylencarbonsäureimiden der Formel I verwendet, in der die Variablen folgende Bedeutung haben:
R, R' unabhängig voneinander Wasserstoff;
C₁-C₃₀-Alkyl, dessen Kohlenstoffkette durch eine oder mehrere Gruppierungen -O-, -S-, -NR¹-, -CO- und/oder -SO₂- unterbrochen sein kann und das durch Cyano, C₁-C₆-Alkoxy, Aryl, das durch C₁-C₁₈-Alkyl oder C₁-C₆-Alkoxy substituiert sein kann, und/oder einen über ein Stickstoffatom gebundenen 5- bis 7-gliedrigen heterocyclischen Rest, der weitere Heteroatome enthalten und aromatisch sein kann, ein- oder mehrfach substituiert sein kann;
C₅-C₈-Cycloalkyl, dessen Kohlenstoffgerüst durch eine oder mehrere Gruppierungen -O-, -S- und/oder -NR¹- unterbrochen und/oder das durch C₁-C₆-Alkyl ein- oder mehrfach substituiert sein kann;
Aryl oder Hetaryl, das durch C₁-C₁₈-Alkyl, C₁-C₆-Alkoxy, Cyano, Halogen, -CONHR², -SO₂R², -SO₂NR²₂ und/oder Aryl- oder Hetarylazo, das jeweils durch C₁-C₁₀-Alkyl, C₁-C₆-Alkoxy oder Cyano substituiert sein kann, ein- oder mehrfach substituiert sein kann;
R¹ Wasserstoff oder C₁-C₆-Alkyl;
R² Wasserstoff; C₁-C₁₈-Alkyl; Aryl oder Hetaryl, das jeweils durch C₁-C₆-Alkyl, C₁-C₆-Alkoxy, Halogen, Hydroxy, Carboxy oder Cyano substituiert sein kann.

3. Verfahren nach Anspruch 1 oder 2, **dadurch gekennzeichnet, daß** man das Lösungsmittel L1 mit einem Lösungsmittel L2 in Kontakt bringt, von dem der Halogenwasserstoff aus dem Lösungsmittel L1 extrahiert wird und das sich nicht mit dem Lösungsmittel L1 mischt und/oder einen höheren Siedepunkt als das Lösungsmittel L1 aufweist und destillativ von diesem trennbar ist.

4. Verfahren nach den Ansprüchen 1 bis 3, **dadurch gekennzeichnet, daß** man als Lösungsmittel L2 Wasser oder wäßrige Lösungen von anorganischen Oxidationsmitteln und/oder anorganischen Basen einsetzt.

5. Verfahren nach Anspruch 3 oder 4, **dadurch gekennzeichnet, daß** man die Halogenierung unter Rühren in einem Zweiphasengemisch aus Lösungsmittel L1 und Lösungsmittel L2 durchführt.

6. Verfahren nach Anspruch 3 oder 4, **dadurch gekennzeichnet, daß** man die Halogenierung in dem Lösungsmittel L1 vornimmt und dieses wiederholt mit dem Lösungsmittel L2 in Kontakt bringt und auf diese Weise den gebildeten Halogenwasserstoff entzieht.

7. Verfahren nach den Ansprüchen 1 bis 6, **dadurch gekennzeichnet, daß** man als Lösungsmittel L1 halogenierte aliphatische oder aromatische oder nitrierte aromatische Kohlenwasserstoffe einsetzt.

8. Verfahren nach den Ansprüchen 1 bis 7, **dadurch gekennzeichnet, daß** man als Lösungsmittel L1 Chloroform, Methylenchlorid oder Chlorbenzol einsetzt.

9. Verfahren nach den Ansprüchen 1 bis 8, **dadurch gekennzeichnet, daß** man als Rylencarbonsäureimide der Formel II Perylen-3,4-dicarbonsäuremonoimide, Terrylen-3,4:11,12-tetracarbonsäuredümide oder Quaterrylen-3,4:13,14-tetracarbonsäurediimide einsetzt.

10. Verfahren nach den Ansprüchen 1 bis 9, **dadurch gekennzeichnet, daß** man als Halogen Brom einsetzt.

11. Verfahren nach den Ansprüchen 1 bis 10, **dadurch gekennzeichnet, daß** man es zur Herstellung von Tribromperylen-3,4-dicarbonsäuremonoimiden, Tetrabromterrylen-3,4:11,12-tetracarbonsäurediimiden und Hexabromquaterrylen-3,4:13,14-tetracarbonsäuredümiden anwendet.

## Claims

1. A process for preparing halogenated rylenecarboximides of the general formula I 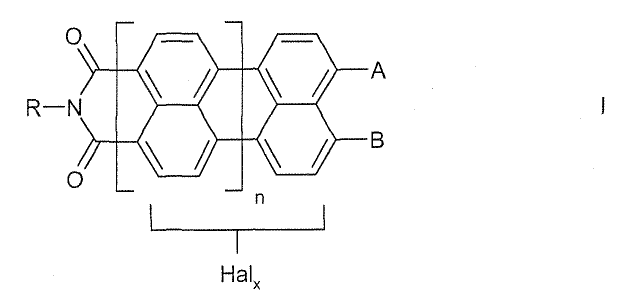 in which the variables are each defined as follows:
A, B together are an imide radical of the formula 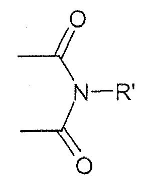 or, in the case that n is 1, A and B are each Hal or one radical is Hal and the other radical is hydrogen;
R, R' are each independently:
hydrogen;
(1) C₁-C₃₀-alkyl whose carbon chain may be interrupted by one or more -O-; -S-, -NR¹-, -N=CR¹-, -C≡C-, -CR¹=CR¹, -CO-, -SO- and/or SO₂- moieties and which may be mono- or polysubstituted by:
(i) C₁-C₁₂-alkoxy, C₁-C₆-alkylthio, -C≡CR¹, -CR¹=CR¹₂, hydroxyl, mercapto, halogen, cyano, nitro, -NR²R³, -NR²COR³, -CONR²R³, -SO₂NR²R³, -COOR², -SO₃R², -PR²R³ and/or -POR²R³;
(ii) aryl or hetaryl, to which may be fused further saturated or unsaturated 5- to 7-membered rings whose carbon skeleton may be interrupted by one or more -O-, -S-, -NR¹-, -N=CR¹-, -CR¹=CR¹-, -CO-, -SO- and/or -SO₂- moieties, where the entire ring system may be mono- or polysubstituted by: C₁-C₁₈-alkyl, C₁-C₁₂-alkoxy, C₁-C₆-alkylthio, -C≡CR¹, -CR¹=CR¹₂, hydroxyl, mercapto, halogen, cyano, nitro, -NR²R³, -NR²COR³, -CONR²R³, -SO₂NR²R³, -COOR², -SO₃R ² -PR²R³, -POR²R³, aryl and/or hetaryl, each of which may be substituted by C₁-C₁₈-alkyl, C₁-C₁₂-alkoxy, hydroxyl, mercapto, halogen, cyano, nitro, -NR²R³, -NR²COR³, -CONR²R³, -SO₂NR²R³, -COOR², -SO₃R², -PR²R³ and/or -POR²R³;
(iii) C₃-C₈-cycloalkyl whose carbon skeleton may be interrupted by one or more -O-, -S-, -NR¹-, -N=CR¹-, -CR¹=CR¹-, -CO-, -SO- and/or -SO₂- moieties and to which may be fused further saturated or unsaturated 5- to 7-membered rings whose carbon skeleton may be interrupted by one or more -O-, -S-, -NR¹-, -N=CR¹-, -CR¹=CR¹-, -CO-, -SO- and/or -SO₂- moieties, where the entire ring system may be mono- or polysubstituted by: C₁-C₁₈-alkyl, C₁-C₁₂-alkoxy, C₁-C₆-alkylthio, -C≡CR¹, -CR¹=CR¹₂, hydroxyl, mercapto, halogen, cyano, nitro, -NR²R³, -NR²COR³, -CONR²R³, -SO₂NR²R³, -COOR², -SO₃R ², -PR²R³ and/or -POR²R³;
(iv) a -U-aryl radical which may be mono- or polysubstituted by the above radicals specified as substituents for the aryl radicals (ii), where U is an -O-, -S-, -NR¹-, -CO-, -SO- or -SO₂- moiety;
(2) C₃-C₈-cycloalkyl, to which may be fused further saturated or unsaturated 5- to 7-membered rings whose carbon skeleton may be interrupted by one or more -O-, -S-, -NR¹-, -N=CR¹-, -CR¹=CR¹-, -CO-, -SO- and/or -SO₂- moieties, where the entire ring system may be mono- or polysubstituted by the (i), (ii), (iii), (iv) radicals, and/or
(v) C₁-C₃₀-alkyl whose carbon chain may be interrupted by one or more -O-, -S-, -NR¹-, -N=CR¹-, -C≡C-, -CR¹=CR¹-, -CO-, -SO- and/or -SO₂- moieties and which may be mono- or polysubstituted by: C₁-C₁₂-alkoxy, C₁-C₆-alkylthio, -C≡CR¹, -CR¹=CR¹₂, hydroxyl, mercapto, halogen, cyano, nitro, -NR²R³, -NR²COR³, -CONR²R³, -SO₂NR²R³, -COOR², -SO₃R ², -PR²R³, -POR²R³, aryl and/or saturated or unsaturated C₄-C₇-cycloalkyl whose carbon skeleton may be interrupted by one or more -O-, -S-, -NR¹-, -N=CR¹-, -CR¹=CR¹-, -CO-, -SO- and/or -SO₂- moieties, where the aryl and cycloalkyl radicals may each be mono- or polysubstituted by C₁-C₁₈-alkyl and/or the above radicals specified as substituents for alkyl;
(3) aryl or hetaryl, to which may be fused further saturated or unsaturated 5- to 7-membered rings whose carbon skeleton may be interrupted by one or more -O-, -S-, -NR¹-, -N=CR¹-, -C≡C-, -CR¹=CR¹-, -CO-, -SO- and/or -SO₂- moieties, where the entire ring system may be substituted by the (i), (ii), (iii), (iv), (v) radicals, and/or aryl- and/or hetarylazo, each of which may be substituted by C₁- C₁₀-alkyl, C₁-C₆-alkoxy and/or cyano;
R¹ is hydrogen or C₁-C₁₈-alkyl, where the R¹ radicals may be the same or different when they occur more than once;
R², R³ are each independently hydrogen;
C₁-C₁₈-alkyl whose carbon chain may be interrupted by one or more -O-, -S-, -CO-, -SO- and/or -SO₂- moieties and which may be mono- or polysubstituted by C₁-C₁₂-alkoxy, C₁-C₆-alkylthio, hydroxyl, mercapto, halogen, cyano, nitro and/or -COOR¹;
aryl or hetaryl, to each of which may be fused further saturated or unsaturated 5- to 7-membered rings whose carbon skeleton may be interrupted by one or more -O-, -S-, -CO- and/or -SO₂- moieties, where the entire ring system may be mono- or polysubstituted by C₁-C₁₂-alkyl and/or the above radicals specified as substituents for alkyl;
Hal is chlorine, bromine or iodine;
n is 1, 2 or 3;
x is from 1 to 8,
by reacting a rylenecarboximide of the general formula II 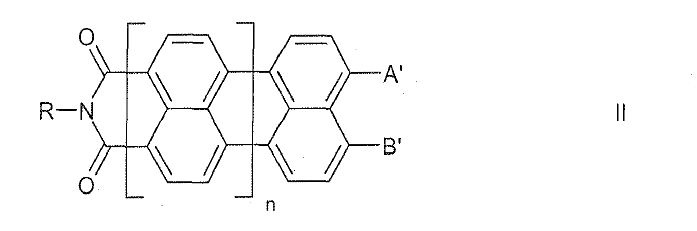 in which the A' and B' radicals together are an imide radical of the formula 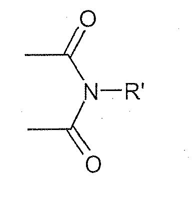 or, in the case that n is 1, are also each hydrogen
with elemental halogen in the presence of an inert organic solvent S1, which comprises continuously withdrawing the hydrogen halide formed in the course of the reaction from the solvent S1.

2. The process according to claim 1, which is used to prepare halogenated rylenecarboximides of the formula I in which the variables are each defined as follows:
R, R' are each independently hydrogen;
C₁-C₃₀-alkyl whose carbon chain may be interrupted by one or more -O-,-S-, -NR¹-, -CO- and/or -SO₂- moieties and which may be mono- or polysubstituted by cyano, C₁-C₆-alkoxy, aryl which may be substituted by C₁-C₁₈-alkyl or C₁-C₆-alkoxy, and/or a 5- to 7-membered heterocyclic radical which is bonded via a nitrogen atom and may comprise further heteroatoms and be aromatic;
C₅-C₈-cycloalkyl whose carbon skeleton may be interrupted by one or more -O-, -S- and/or -NR¹- moieties and/or which may be mono- or polysubstituted by C₁-C₆-alkyl;
aryl or hetaryl, each of which may be mono- or polysubstituted by C₁-C₁₈-alkyl, C₁-C₆-alkoxy, cyano, halogen, -CONHR², -SO₂R², -SO₂NR²₂ and/or aryl- or hetarylazo, each of which may be substituted by C₁-C₁₀-alkyl, C₁-C₆-alkoxy or cyano;
R¹ is hydrogen or C₁-C₆-alkyl;
R² is hydrogen; C₁-C₁₈-alkyl; aryl or hetaryl, each of which may be substituted by C₁-C₆-alkyl, C₁-C₆-alkoxy, halogen, hydroxy, carboxy or cyano.

3. The process according to claim 1 or 2, wherein the solvent S1 is contacted with a solvent S2 by which the hydrogen halide is extracted from the solvent S1 and which does mix with the solvent S1 and/or has a higher boiling point than the solvent S1 and can be separated from it by distillation.

4. The process according to claims 1 to 3, wherein the solvent S2 used is water or an aqueous solution of inorganic oxidizing agents and/or inorganic bases.

5. The process according to claim 3 or 4, wherein the halogenation is carried out with stirring in a biphasic mixture of solvent S1 and solvent S2.

6. The process according to claim 3 or 4, wherein the halogenation is undertaken in the solvent S1 which is contacted repeatedly with the solvent S2 and the hydrogen halide formed is removed in this way.

7. The process according to claims 1 to 6, wherein the solvent S1 used is a halogenated aliphatic or aromatic or nitrated aromatic hydrocarbon.

8. The process according to claims 1 to 7, wherein the solvent S1 used is chloroform, methylene chloride or chlorobenzene.

9. The process according to claims 1 to 8, wherein the rylenecarboximides of the formula II used are perylene-3,4-dicarboximides, terrylene-3,4:11,12-tetracarboximides or quaterrylene-3,4:13,14-tetracarboximides.

10. The process according to claims 1 to 9, wherein the halogen used is bromine.

11. The process according to claims 1 to 10, which is used to prepare tribromoperylene-3,4-dicarboximides, tetrabromoterrylene-3,4:11,12-tetra-carboximides and hexabromoquaterrylene-3,4:13,14-tetracarboximides.

## Revendications

1. Procédé de préparation d'imides d'acide rylène carboxylique halogénés de formule générale I 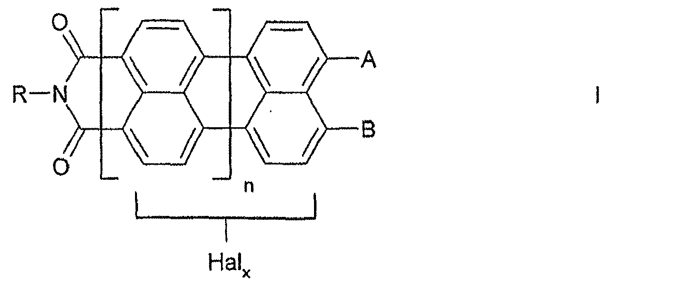 dans laquelle les variables ont la signification suivante :
A, B forment ensemble, un radical imide de formule 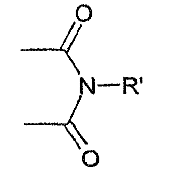 ou pour le cas où n est égal à 1, A et B signifient tous deux Hal ou un radical signifie Hal et l'autre radical l'hydrogène;
R, R' indépendamment l'un de l'autre sont:
l'hydrogène;
(1) un alkyle en C₁-C₃₀ dont la chaîne de carbone peut être interrompue par un ou plusieurs groupements -O-, -S-, -NR¹-, -N=CR¹-, -C≡C-, -CR¹=CR¹-, -CO-, -SO- et/ou -SO₂- et qui peut être une ou plusieurs fois substitué par :
(i) un alcoxy en C₁-C₁₂, un alkylthio en C₁-C₆, -C≡CR¹, -CR¹=CR¹₂, un hydroxy, un mercapto, un halogène, un cyano, un nitro, -NR²R³, -NR²COR³, -CONR²R³, -SO₂NR²R³, -COOR², -SO₃R², PR²R³ et/ou -POR²R³;
(ii) un aryle ou un hétaryle, auquel on peut fusionner d'autres cycles de 5 à 7 membres saturés ou insaturés dont le squelette de carbone peut être interrompu par un ou plusieurs groupements -O-, -S-, -NR¹-, -N=CR¹-, -CR¹=CR¹-, -CO-, -SO- et/ou -SO₂-, tout le système de cycle pouvant être une ou plusieurs fois substitué par : un alkyle en C₁-C₁₈, un alcoxy en C₁-C₁₂, un alkylthio en C₁-C₆, -C≡CR¹-, -CR¹=CR¹₂, un hydroxy, un mercapto, un halogène, un cyano, un nitro, -NR²R³, -NR²COR³,
-CONR²R³, -SO₂NR²R3, -COOR², SO₃R², -PR²R³, -POR²R³, un aryle et/ou un hétaryle, qui peut être chaque fois substitué par un alkyle en C₁-C₁₈, un alcoxy en C₁-C₁₂, un hydroxy, un mercapto, un halogène, un cyano, un nitro, -NR²R³, -NR²COR³, -CONR²R³, -SO₂NR²R³, -COOR², -SO₃R², -PR²R³ et/ou -POR²R³;
(iii) un cycloalkyle en C₃-C₈ dont le squelette de carbone peut être interrompu par un ou plusieurs groupements -O-, -S-, -NR¹-, -N=CR¹-, -CR¹=CR¹-, -CO-, -SO- et/ou -SO₂- et auquel d'autres cycles de 5 à 7 membres saturés ou insaturés, dont le squelette de carbone peut être interrompu par un ou plusieurs groupements -O-, -S-, -NR¹-, -N=CR¹-, -CR¹=CR¹-, -CO-, -SO- et/ou -SO₂-, peuvent être fusionnés, tout le système de cycle pouvant être une ou plusieurs fois substitué par : un alkyle en C₁-C₁₈, un alcoxy en C₁-C₁₂, un alkylthio en C₁-C₆, -C≡CR¹, -CR₁=CR¹₂, un hydroxy, un mercapto, un halogène, un cyano, un nitro, -NR²R³, -NR²COR³, -CONR²R³, -SO₂NR²R³, -COOR², -SO₃R², -PR²R³ et/ou -POR²R³;
(iv) un radical -U-aryle qui peut être substitué une ou plusieurs fois par les radicaux mentionnés ci-dessus comme substituants pour les radicaux aryle (ii), U signifiant un groupement -O-, -S-, -NR¹-, -CO-, -SO- ou -SO₂-;
(2) un cycloalkyle en C₃-C₈, auquel d'autres cycles de 5 à 7 membres saturés ou insaturés dont le squelette de carbone peut être interrompu par un ou plusieurs groupements -O-, -S-, -NR¹-, -N=CR¹-, -CR¹=CR¹-, -CO-, -SO- et/ou -SO₂-, peuvent être fusionnés, tout le système de cycle pouvant être une ou plusieurs fois substitué par : les radicaux (i), (ii), (iii), (iv) et/ou
(v) un alkyle en C₁-C₃₀ dont la chaîne de carbone peut être interrompue par un ou plusieurs groupements -O-, -S-, -NR¹-, -N=CR¹-, -C≡C-, -CR¹=CR¹-, -CO-, -SO- et/ou -SO₂- et qui peut être une ou plusieurs fois substitué par : un alcoxy en C₁-C₁₂, un alkylthio en C₁-C₆, -C≡CR¹, -CR¹=CR¹₂, un hydroxy, un mercapto, un halogène, un cyano, un nitro, -NR²R³, -NR²COR³, -CONR²R³, -SO₂NR²R³, -COOR², -SO₃R²,-PR²R³, -POR²R³, un aryle et/ou un cycloalkyle en C₄-C₇ saturé ou insaturé dont le squelette de carbone peut être interrompu par un ou plusieurs groupements -O-, -S-, -NR¹-, -N=CR¹-, -CR¹=CR¹-, -CO-, -SO- et/ou -SO₂-, les radicaux aryle et cycloalkyle pouvant être substitués chaque fois une ou plusieurs fois par un alkyle en C₁-C₁₈ et/ou par les radicaux mentionnés ci-dessus comme substituants pour alkyle;
(3) un aryle ou hétaryle, auquel d'autres cycles de 5 à 7 membres saturés ou insaturés dont le squelette de carbone peut être interrompu par un ou plusieurs groupements -O-, -S-, -NR¹-, -N=CR¹-, -C≡C-, -CR¹=CR¹-, -CO-, -SO- et/ou -SO₂-, peuvent être fusionnés, tout le système de cycle pouvant être substitué par les radicaux (i), (ii), (iii), (iv), (v) et/ou aryle et/ou hétarylazo, qui peut chaque fois être substitué par un alkyle en C₁-C₁₀, un alcoxy en C₁-C₆ et/ou un cyano;
R¹ est l'hydrogène ou un alkyle en C₁-C₁₈, les radicaux R¹ pouvant être identiques ou différents, lorsqu'ils apparaissent plusieurs fois;
R² R³ indépendamment l'un de l'autre sont l'hydrogène;
un alkyle en C₁-C₁₈ dont la chaîne de carbone peut être interrompue par un ou plusieurs groupements -O-, -S-, -CO-, -SO- et/ou -SO₂- et qui peut être une ou plusieurs fois substitué par un alcoxy en C₁-C₁₂, un alkylthio en C₁-C₆, un hydroxy, un mercapto, un halogène, un cyano, un nitro et/ou -COOR¹;
un aryle ou un hétaryle, auquel d'autres cycles de 5 à 7 membres saturés ou insaturés dont le squelette de carbone peut être interrompu par un ou plusieurs groupements -O-, -S-, -CO- et/ou -SO₂-, peuvent être fusionnés, tout le système de cycle pouvant être une ou plusieurs fois substitué par un alkyle en C₁-C₁₂ et/ou par les radicaux cités ci-dessus comme substituants d'alkyle;
Hal est chlore, brome ou iode;
n 1, 2 ou 3;
x 1 à 8,
par réaction d'un imide d'acide rylène carboxylique de formule générale II 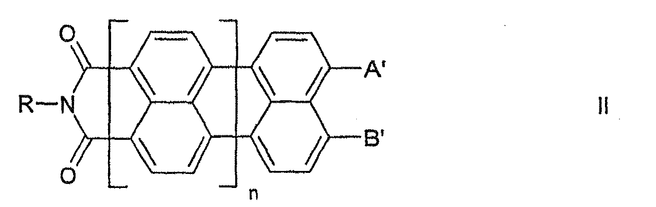 dans laquelle les radicaux A' et B' forment ensemble un radical imide de formule 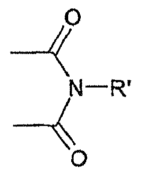 ou pour le cas où n est égal à 1, tous deux signifient également l'hydrogène,
avec un halogène élémentaire en présence d'un solvant organique inerte L1, **caractérisé en ce qu'**on prélève en continu du solvant L1 l'halogénure d'hydrogène qui se forme lors de cette réaction.

2. Procédé selon la revendication 1, **caractérisé en ce qu'**on l'utilise lors de la préparation d'imides d'acide rylène carboxylique halogénés de formule I, dans laquelle les variables ont la signification suivante :
R, R' indépendamment l'un de l'autre, sont l'hydrogène;
un alkyle en C₁-C₃₀ dont la chaîne de carbone peut être interrompue par un ou plusieurs groupements -O-, -S-, -NR¹-, -CO- et/ou -SO₂- et qui peut être une ou plusieurs fois substitué par un cyano, un alcoxy en C₁-C₆, un aryle, lequel peut être substitué par un alkyle en C₁-C₁₈ ou un alcoxy en C₁-C₆, et/ou par un radical hétérocyclique de 5 à 7 membres lié par un atome d'azote, et qui peut contenir d'autres hétéroatomes et être aromatique;
un cycloalkyle en C₅-C₈ dont la structure de carbone peut être interrompue par un ou plusieurs groupements -O-, -S- et/ou -NR¹- et/ou qui peut être une ou plusieurs fois substitué par un alkyle en C₁-C₆,
un aryle ou hétaryle qui peut être une ou plusieurs fois substitué par un alkyle en C₁-C₁₈, un alcoxy en C₁-C₆, un cyano, un halogène, -CONHR², -SO₂R², -SO₂NR²₂ et/ou un aryl- ou hétarylazo, qui peut être chaque fois substitué par un alkyle en C₁-C₁₀, un alcoxy en C₁-C₆ ou un cyano;
R¹ est l'hydrogène ou un alkyle en C₁-C₆;
R² est l'hydrogène; un alkyle en C₁-C₁₈; un aryle ou un hétaryle, qui peut être substitué chaque fois par un alkyle en C₁-C₆, un alcoxy en C₁-C₆, un halogène, un hydroxy, un carboxy ou un cyano.

3. Procédé selon la revendication 1 ou 2, **caractérisé en ce qu'**on met en contact le solvant L1 avec un solvant L2, d'où l'halogénure d'hydrogène est extrait du solvant L1 et qui ne se mélange pas avec le solvant L1 et/ou présente un point d'ébullition supérieur au solvant L1 et dont on peut le séparer par distillation.

4. Procédé selon les revendications 1 à 3, **caractérisé en ce qu'**on utilise comme solvant L2 l'eau ou des solutions aqueuses d'agents d'oxydation inorganiques et/ou de bases inorganiques.

5. Procédé selon la revendication 3 ou 4, **caractérisé en ce qu'**on effectue l'halogénation sous agitation dans un mélange à deux phases hors du solvant L1 et du solvant L2.

6. Procédé selon la revendication 3 ou 4, **caractérisé en ce qu'**on entreprend l'halogénation dans le solvant L1 et qu'on met celui-ci à nouveau en contact avec le solvant L2 et de cette manière, on soutire l'halogénure d'hydrogène formé.

7. Procédé selon les revendications 1 à 6, **caractérisé en ce qu'**on utilise comme solvant L1 des hydrocarbures halogénés aliphatiques ou aromatiques ou aromatiques nitrités.

8. Procédé selon les revendications 1 à 7, **caractérisé en ce qu'**on utilise comme solvant L1 le chloroforme, le chlorure de méthylène ou le chlorobenzène.

9. Procédé selon les revendications 1 à 8, **caractérisé en ce qu'**on utilise comme imides d'acide rylène carboxylique de formule II, des mono-imides d'acide pérylène-3,4-dicarboxylique, des diimides d'acide terylène-3,4:11,12-tétracarboxylique ou des diimides d'acide quaterrylène-3,4:13,14-tétra-carboxylique.

10. Procédé selon les revendications 1 à 9, **caractérisé en ce qu'**on utilise comme halogène le brome.

11. Procédé selon les revendications 1 à 10, **caractérisé en ce qu'**on utilise pour la préparation de mono-imides d'acide tribromopérylène-3,4-dicarboxylique, des diimides d'acide tétrabromoterrylène-3,4:11,12-tétracarboxylique et des diimides d'acide hexabromoquaterrylène-3,4:13,14-tétracarboxylique.
